# EUROPEAN PATENT APPLICATION

(11) **EP 2 662 359 A1**
(43) Date of publication of application: **13.11.2013**
(21) Application number: 12167189.5
(22) Date of filing: 08.05.2012
(51) Int. Cl.: C07D 231/12, H01G 9/20, H01M 10/39, B01D 53/14

(54) **Ionic Liquids, Method for manufacturing thereof, and Electrochemical Devices Comprising the Same**

(71) Applicant: SOLVAY SA, 1120 Bruxelles (BE)
(72) Inventor: Braun, Max Josef, 30900 Wedemark (DE); Fischer, Reiner, 38173 Dettum (DE)
(74) Representative: Mross, Stefan P.M.

(57) **Abstract**

The present invention concerns ionic liquids comprising at least one pyrazolium cation which are substituted by at least one fluorine atom and at least one anion obtainable from 4-ethoxy-1,1,1-trifluoro-3-butene-2-one (ETFBO). The ionic liquids according to the present invention can be used as electrolytes for electrochemical and/or optoelectronic devices.

## Description

The present invention relates to an ionic liquid, and a method for producing the same. The present invention further concerns an electrolyte comprising ionic liquid and an electrochemical device, in particular a battery and a solar cell, comprising the same.

Ionic liquids have been used for a variety of industrial applications, for example as solvents, catalysts, electrically conducting fluids, sealants, surfactants and dispersing agents.

One of the application fields for ionic liquid is as electrolyte for electrochemical devices, such as batteries and solar cells, and/or optoelectronic devices. Particularly, ionic liquids have been investigated as safe electrolytes for high energy density lithium ion battery.

A publication from Chai et al. (Ether-functionalized pyrazolium ionic liquids as new electrolytes for lithium battery, Electrochimica Acta, 66, pp. 67-74 (2012)) discloses two functionalized ionic liquids based on pyrazolium cations and bis(trifluoromethylsulfonyl)imide anions (TFSI⁻) as indicated below and its physicochemical and electrochemical properties along with their performances as electrolytes for lithium battery.

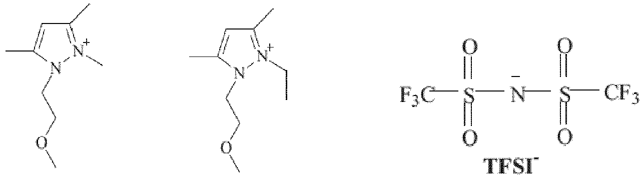

On the other hand, ionic liquids are also candidates to replace volatile organic solvents present in solar cells, in particular dye-sensitized solar cells. International publication No. WO 2007/093961 A1 discloses electrolytes comprising tetracyanoborate and an organic cation as components of electrolytes in electrochemical and/or optoelectronic devices, in particular solar cells, the ionic liquid having low viscosity. It can be used as electrolyte in the absence of solvents, remaining stable in solar cells even after certain prolonged thermal stress.

### DESCRIPTION OF THE INVENTION

The objective of the present invention is to provide novel ionic liquids having particularly advantageous properties, which can be suitably used, in particular for an electrolyte for electrochemical devices and/or optoelectronic devices. The ionic liquids according to the present invention may exhibit a high stability and/or dissolve lithium salts such as LiPF₆ and LiPO₂F₂ in higher amounts than the known ionic liquids.

The present invention therefore relates to ionic liquids comprising a) at least one pyrazolium cation which is substituted by at least one fluorine atom, and b) at least one anion (An). Indeed, it has been surprisingly found that the ionic liquids according to the present invention display a high solubility of lithium salts in general and dissolve better the entire mixtures of additives such as monofluoroethylene carbonate (F1EC) and ethylene carbonate (EC), and these advantages can be extended to inorganic as well as organic fluorine-containing electrolyte mixtures. Besides that, due to its melting point of which is generally no more than 60°C, the ionic liquids according to the present invention can contribute significantly to improve safety aspects of the final battery.

Further, the present invention provides electrolytes comprising the ionic liquid according to the present invention. Still further, the present invention also provides electrochemical and/or optoelectronic devices comprising the electrolyte according to the present invention.

### DETAILED DESCRIPTION OF THE INVENTION

In the present invention, an ionic liquid is understood to denote, in particular, a salt which comprises an electrochemically neutral pair of ions in the liquid state whose melting point is usually below 100°C.

In the present invention, an alkyl group is understood to denote, in particular, a straight chain or branched chain hydrocarbon groups usually having from 1 to 6 carbon atoms or cyclic hydrocarbon groups usually having from 3 to 6 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In the present invention, an ionic liquid comprises at least one anion and at least one pyrazolium cation substituted by at least one fluorine atom. The substitution by at least one fluorine atom is understood to denote, in particular, that at least one hydrogen attached directly to pyrazole moiety and/or at least one hydrogen attached to an optional substituting group attached to the pyrazole moiety is substituted by a fluorine atom. Preferably, the pyrazolium cation which is substituted by at least one fluorine atom has a fluorinated alkyl group attached to pyrazole moiety of the pyrazolium cation.

In one embodiment, the pyrazolium cation has the formula (A) denoted as below.

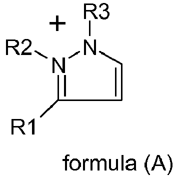

In the formula (A), R1 is a fluorinated alkyl group. In the present invention, "fluorinated" is understood to denote to encompass both meanings of "partially fluorinated" and "perfluorinated." The fluorinated alkyl group is preferably selected from CF₃, CF₂H and CF₂Cl, and more preferably CF₃. In the formula (A), R2 and R3 are independently selected from and are hydrogen or an optionally fluorinated Cl to C6 alkyl group, preferably from an optionally fluorinated methyl group, ethyl group and butyl group, more preferably from a methyl group, an ethyl group and a butyl group.

In preferred embodiments, R1, R2 and R3 have the following meaning :

| R1 | R2 | R3 |
|---|---|---|
| CF₃ | CH₃ | CH₃ |
| CF₂H | CH₃ | CH₃ |
| CF₂Cl | CH₃ | CH₃ |
| CF₃ | CH₃ | CH₂CH₃ |
| CF₂H | CH₃ | CH₂CH₃ |
| CF₂Cl | CH₃ | CH₂CH₃ |
| CF₃ | CH₂CH₃ | CH₃ |
| CF₂H | CH₂CH₃ | CH₃ |
| CF₂Cl | CH₂CH₃ | CH₃ |
| CF₃ | CH₂CH₃ | CH₂CH₃ |
| CF₂H | CH₂CH₃ | CH₂CH₃ |
| CF₂Cl | CH₂CH₃ | CH₂CH₃ |

Preferably, R1 is CF₃, and R2 and R3 are CH₃.

In the present invention, an anion may be denoted as "An". In a particular embodiment, An is selected from the group consisting of halides, BF₄⁻, B(CN)₄⁻, CH₃BF₃⁻, CH₂CHBF₃⁻ , CF₃BF₃⁻ , PF₆⁻ , CF₃CO₂⁻ , CF₃SO₃⁻ , (CF₃SO₂)₂N⁻, (CF₃SO)(CF₃CO)N⁻, (CN)₂N⁻, C(CN)₃⁻, SCN⁻, SeCN⁻, CuCl₂⁻, AlCl₄⁻, (CF₃)₂CFSO₃⁻, P(CN)ₚX₆₋ₚ (wherein p is an integer selected from between 1 and 6), (CF₃)₂CFCO₂⁻, and any combination thereof. More preference as An is given to CF₃SO₃⁻.

The present invention also concerns methods of manufacturing an ionic liquid according to the present invention.

In one embodiment, the method of manufacturing an ionic liquid comprises a step of contacting a compound having the formula Alk-An, wherein Alk is an optionally fluorinated C 1 to C6 alkyl group and An has the meaning given above, with a solution of the pyrazole compound having formula (a-1) or formula (a-2) below, or the mixture thereof :

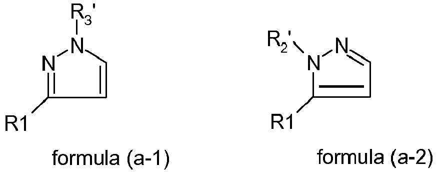

In the formula (a-1), R1 has the same meaning as denoted above and is a fluorinated alkyl group preferably selected from CF₃, CF₂H and CF₂Cl, and R2' and R3' are independently hydrogen or an optionally fluorinated Cl to C6 alkyl group, preferably selected from hydrogen, methyl group, ethyl group and butyl group.

In a further embodiment, wherein R3' is hydrogen, the method of manufacturing the ionic liquids according to the present invention comprises a further step of contacting alkyl halide having the formula R3-halide with the pyrazole compound, before conducting the step of contacting Alk-An with a solution of the pyrazole compound having formula (a-1); R3 in R3-halide has the same meaning given above.

In the present invention, the pyrazole compound having formula (a-1) may be obtained by, but not limited to, the process disclosed in International Publication No. WO 2010/037688 A1 . For instance, the pyrazole compound having formula (a-1) is obtainable by the process comprising (a) step of adding an acid halide of formula R1-C(O)-X, wherein R1 is a halogenated alkyl group and X = fluorine, chlorine or bromine, to a vinyl either of formula CH₂=CH-OR3', wherein R3' is hydrogen or an optionally fluorinated C1 to C6 alkyl group, to produce an addition product, and (b) step of reacting said addition product with hydrazine or hydrazine derivatives. More details are available from WO 2010/037688 A1.

The present invention also relates to an electrolyte comprising the ionic liquid according to the present invention. In a certain embodiment, the electrolyte comprises 0.1 to 80 vol. % of the ionic liquid according to the present invention. Preferably, the electrolyte comprises 2 to 50 vol. %, more preferably 8 to 45 vol. %, most preferably 10 to 40 vol. % of the ionic liquid of the present invention. Here, although the volume percentages are given to refer to the volume at 25°C, if a given compound or composition is not liquid at this temperature, the volume may be measured at the lowest temperature at which volume can be determined, typically at the melting point of the compound or composition.

In a certain other embodiment, the electrolyte according to the present invention further comprises at least one conducting salt. The conducting salt is preferably selected from, but is not limited to, lithium salts. The lithium salts can be selected from, but are not limited to, the group consisting of LiPF₆, LiPO₂F₂, LiBF₄, LiB(CN)₄, LiAsF₆, LiClO₄, LiBOB (lithium bisoxalatoborate), LiFSI (lithium bis(fluorosulfonyl)imidide), Li[N(SO₂CₙF₂ₙ₊₁)(SO₂CₘF₂ₘ₊₁)] (wherein n and m are independently integers selected from between 1 and 10, such as LiTFSI (lithium bis(trifluoromethylsulfonyl)imidide) or LiN(CF₃SO₂)₂), LiBeti (lithium bis(perfluoroethylsulfonyl)imidide), LiODBF (LiBF₂C₂O₄), LiB(C₆H₅), LiCF₃SO₃, LiC(CF₃SO₂)₃, and any combination thereof. Preferred lithium salts are LiPF₆, LiPO₂F₂, and LiTFSI. The electrolyte according to the present invention comprises preferably 0.1 to 10 mol/L of the conducting salts.

In a still another embodiment, the electrolyte according to the present invention comprises at least one further ionic liquid with iodide as anion. The cation of the at least one further ionic liquid is selected from organic cations, preferably from the group consisting of hydroxonium, oxonium, ammonium, amidinium, phosphonium, uronium, thiouronium, guanidinium, sulfonium, phospholium, phosphorolium, iodonium, carbonium cations; heterocyclic cations such as pyridinium, quinolinium, isoquinolinium, imidazolium, pyrazolium, imidazolinium, triazolium, pyridazinium, pyrimidinium, pyrrolidinium, thiazolium, oxazolium, pyrazinium, piperazinium, piperidinium, pyrrolium, pyrizinium, indolium, quinoxalinium, thiomorpholinium, morpholinium, and indolinium cations; and any combination thereof. More preferably, the cation is selected from the group consisting of unsubstituted or substituted imidazoliums such as the di-, tri-, tetra- and penta-alkyl imidazoliums, quaternary ammoniums, unsubstituted or substituted piperidiniums such as dialkylpiperidiniums, unsubstituted or substituted pyrrolidiniums such as dialkylpyrrolidiniums, unsubstituted or substituted pyrazoliums such as dialkylpyrazoliums or the pyrazolium having the formula (A), unsubstituted or substituted pyridiniums such as alkylpyridiniums, phosphoniums such as tetraalkylphosphoniums, sulfoniums such as trialkylsulfoniums, and any combination thereof, most preferably, selected from piperidiniums such as dialkylpiperidiniums; quaternary ammoniums such as quaternary ammoniums bearing four alkyl groups; imidazoliums such as di-, tri-, tetra- and penta-substituted imidazoliums such as di-, tri-, tetra-, and penta-alkyl imidazoliums; and any combination thereof.

The examples of the further ionic liquid include 1-butyl-3-methylimidazolium iodide, p-methylbutylpyridinium iodide, 1-ethyl-3-methylimidazolium iodide, and 1-methyl-3-propylimidazolium iodide.

The present invention still further relates to electrochemical devices and/or optoelectronic devices comprising the ionic liquid or the electrolyte according to the present invention. The electrochemical device and optoelectronic device, respectively, can be selected from batteries and solar cells. Preferred devices are selected from the group consisting of lithium ion battery, dye-sensitized solar cell (DSSC) or organic photovoltaic (OPV).

The devices according to the present invention may be manufactured in the same manner as the corresponding device of the prior art by simply replacing the electrolyte during the manufacture by the electrolyte according to the present invention. A non-limiting example is the manufacture of dye-sensitized solar cells, as disclosed in the examples of WO 2007/093961 A1.

The novel ionic liquids according to the present invention can also be used in other applications than electrochemical and/or optoelectronic devices. Such other applications include, but are not limited to, the use as solvents, catalysts, electrically conducting fluids, sealants, surfactants or dispersing agents. Therefore, the present invention also concerns the use of an ionic liquid comprising at least one pyrazolium cation which is substituted by at least one fluorine atom, preferably having the formula (A), and at least one anion denoted as An, as solvents, catalysts, electrically conducting fluids, sealants, surfactants or dispersing agents. 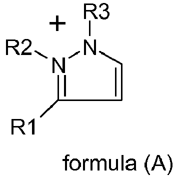 wherein, R1, R2 and R3 have the same meanings described above.

The ionic liquids according to the present invention can also be used as solvent for separating hydrogen halide from a gas mixture containing hydrogen halide and at least one other gas, preferably COF₂.

### BRIEF DESCRIPTION OF THE DRAWING(S)

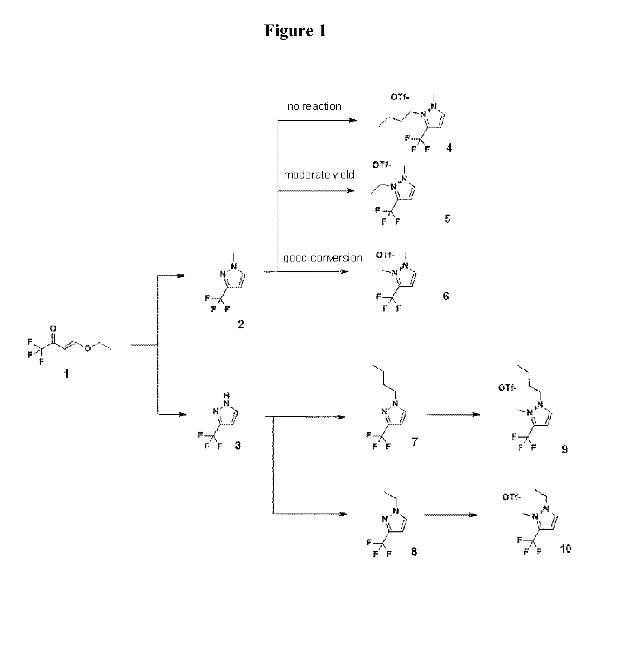

Figure 1 shows a reaction scheme for the synthesis of compounds according to the Examples.

The following examples explain the invention in further detail without the intention to limit.

### Examples

### Example 1 : Preparation of ionic liquid according to figure 1

4-ethoxy-1,1,1-trifluoro-3-butene-2-one (ETFBO) was converted to the ionic liquid precursors, i.e. pyrazoles 2 and 3 in figure 1, by a conversion of ETFBO with hydrazine and methylhydrazine as described in International Publication No. WO 2010/037688 A1. Downstream chemistries of the pyrazoles 2 and 3 were performed as below.

### Example 1-1: Preparation of ionic liquid 1-methyl-2-ethyl-3-(trifluoromethyl)-1H-pyrazolium triflate (compound 5 in figure 1)

To a stirred solution of 3.8 g (25.3 mmol) 1-methyl-3-(trifluoromethyl)-1H-pyrazole **(2)** in dichloromethane (75 ml) was added 3.42 ml (26 mmol) of ethyltriflate. After heating the mixture to reflux for 6 hours the low boiling components were removed under reduced pressure to yield 2.4 g (29 %) of the ionic liquid **5** as amber coloured oil.

### Example 1-2: Preparation of ionic liquid 1,2-dimethyl-3-(trifluoromethyl)-1H-pyrazolium triflate (compound 6 in figure 1)

To a stirred solution of 11.5 g (76,6 mmol) 1-methyl-3-(trifluoromethyl)-1H-pyrazole **(2)** in dichloromethane (100 ml) was added 8.75 ml (79.8 mmol) of methyltriflate. The solvent was removed after stirring overnight at ambient temperature. Crystallization from MTBE gave 19 g (79 %) of the ionic liquid **6** as colourless solid.

### Example 1-3: Preparation of ionic liquid 1-butyl-3-(trifluoromethyl)-1H-pyrazole (compound 7 in figure 1)

To a stirred solution of 10 g (73.5 mmol) 3-(trifluoromethyl)-1H-pyrazole **(3)** in DMSO (150 ml) were added 9.9 g (88.2 mmol) of potassium tert.-butoxide portion wise and 1.2 g of potassium iodide. After stirring for 5 minutes drop wise addition of 16 ml of 1-bromobutane followed by stirring overnight were conducted. Water was added afterwards and the pH value was adjusted to 3 by using 1N HCl. The mixture was diluted with MTBE and extracted two more times with MTBE. The combined organic phase was washed with water and brine yielding 11.6 g (82 %) of the ionic liquid **7** accompanied by 11 % of the corresponding isomer.

### Example 1-4: Preparation of ionic liquid 1-ethyl-3-(trifluoromethyl)-1H-pyrazole (compound 8 in figure 1)

To a stirred solution of 10 g (73.5 mmol) 3-(trifluoromethyl)-1H-pyrazole **(3)** in DMSO (150 ml) were added 9.9 g of potassium tert.-butoxide portion wise. After stirring for 5 minutes drop wise addition of 12 ml of iodoethane followed by stirring overnight were conducted. Water was added afterwards and the pH value was adjusted to 3 by using 1N HCl. The mixture was diluted with MTBE and extracted two more times with MTBE. The combined organic phase was washed with Na2S2O3-solution, water and brine yielding 8 g (67 %) of the ionic liquid **8**.

### Example 1-5: Preparation of ionic liquid 1-butyl-2-methyl-3-(trifluoromethyl)-1H-pyrazolium triflate (compound 9 in figure 1)

To a stirred solution of 11 g (57.2 mmol) 1-butyl-3-(triftuoromethyl)-1H-pyrazole (7) [containing about 11 % of the 2-butyl-isomer] in dichloromethane (200 ml) was added 6.6 ml (59 mmol) of methyltriflate. The solvent was removed after stirring overnight at ambient temperature. Crystallization of the residue from MTBE and drying of the resulting precipitate in vacuo gave 14 g (69 %) of the ionic liquid **9** as beige solid, 89 % by HPLC.

### Example 1-6: Preparation of ionic liquid 1-ethyl-2-methyl-3-(trifluoromethyl)-1H-pyrazolium triflate (compound 10 in figure 1)

To a stirred solution of 7.5 g (45,7 mmol) 1-ethyl-3-(triftuoromethyl)-1H-pyrazole **(8)** in dichloromethane (150 ml) was added 5.1 ml (46.5 mmol) of methyltriftate. The solvent was removed after stirring overnight at ambient temperature. Crystallization of the residue from MTBE and drying of the resulting precipitate in vacuo gave 7.7 g (51 %) of the ionic liquid **10** as colourless solid.

### Example 2: Up-scale preparation of 1,2-dimethyl-3-(trifluoromethyl)-1H-pyrazolium triflate

*Example 2-1 : Preparation of methyl-3-(trtfluoromethyl)-pyrazole mixture*

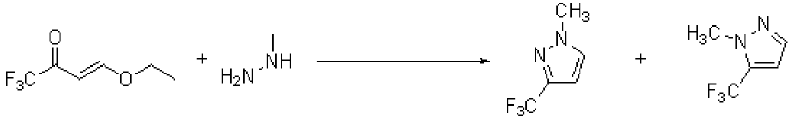

ETFBO (270 g, 1.61 mol, 1.00 eq) was dissolved in methanol (750 mL). The solution was cooled in an ice/water-bath and methyl hydrazine (88.3 mL, 77.7 g, 1.69 mol, 1.05 eq) was added dropwise within 20 minutes. The mixture was heated to 55°C and stirred at this temperature for 20 h in dry air (drying tube). Successively the solvent was removed and the residue distilled under vacuum :
1^{st} Fraction : 150 mbar, 130°C bath temperature, 73~85°C, mixture of isomers, 129 g.
2^{nd} Fraction : 70 mbar, 150°C bath temperature, 75~85°C, 1-methyl-3-(trifluoromethyl)-1H-pyrazole, 24 g. Overall yield : 153 g (1.02 mol) 63 %.
Example 2-2 : Preparation of 1,2-dimethyl-3-(trifluoromethyl)-1H-pyrazolium triflate

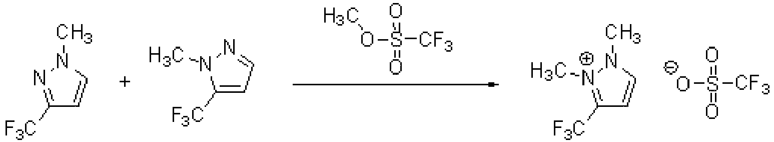

*The methyl-3-(trifluoromethyl)-pyrazole mixture* (mixture of isomers, 179 g, 1.19 mol, 1.00 eq) was dissolved in 2 liters of dichloromethane. Methyltriflate (151 mL, 202 g, 1.23 mol, 1.03 eq) was added and the mixture stirred for 16 hours at room temperature. Successively the solvent was removed and the residue dried under vacuum at 50°C. MTBE (1 liter) was added and the mixture was stirred at room temperature for 20 minutes. The precipitating solid was filtered of with a frit, washed thoroughly with MTBE and dried under vacuum. The product was obtained as a white solid in 230 g (732 mmol, 62 %) yield. Not reacted starting material can be re-isolated from the filtrate.
**m.p.** = 64°C
**¹H-NMR** (500 MHz, DMSO-d6) δ = 4.16 (s, 3 H), 4.21 (s, 3 H), 7.56 (d, ³*J*= 3.0 Hz, 1 H), 8.76 (d, ³*J*= 3 Hz, 1 H) ppm.
**¹³C{¹H}-NMR** (125 MHz, DMSO-d6) δ = 36.1 (CH₃), 37.9 (CH₃), 108.3 (CH), 117.8 (q, C-CF₃), 120.6 (q, SO₃-CF₃), 134.8 (q, *C-*CF₃), 138.1 (CH) ppm.

### Example 3 : Preparation of electrolyte comprising the ionic liquid and a lithium conducting salt

The electrolyte is formulated by dissolving LiPF₆ (lithium hexafluorophosphate) in an equimolar mixture of ionic liquid prepared in the Example 1-2 or 2-2, i.e. 1,2-dimethyl-3-(trifluoromethyl)-1H-pyrazolium triflate.

### Example 4 : Fabrication of lithium ion battery comprising the electrolyte according to the present invention

The following items are stacked from the bottom of the casing of the button battery cell.
1) a negative electrode, the active material of which is Li₄Ti₅O₁₂, but any other negative electrode active material selected from conventional active materials used in the art for a negative electrode can be used.
2) the electrolyte as prepared in the Example 3.
3) a separator which is a microporous membrane in polyolefin, more specifically a microporous membrane in polypropylene.
4) a positive electrode, the active material of which is LiNi₀.₅Mn₁.₅O₄, but a disc of lithium or of any other type of positive electrode active material selected from conventional active materials used in the art for a positive electrode can be used.
5) a stainless steel disc or shim, skid.
6) a stainless steel lid and a bottom in stainless steel.
7) a stainless steel spring and a polypropylene gasket, joint.

The stainless steel casing is then closed with a crimping machine, making it airproof. The button cells undergoes cycling operations, i.e. charging and discharging operations in order to evaluate the performance of the electrolyte according to the present invention.

### Example 5 : Fabrication of dye-sensitized solar cells comprising the ionic liquid according to the present invention

TiO₂ electrodes are prepared of transparent layer in accordance with the literature, such as [Yum, J. H.; Jang, S. R.; Humphry-Baker, R.; Gratzel, M.; Cid, J. J.; Torres, T.; Nazeeruddin, M. K. Langmuir 2008, 24, 5636], and the TiO₂ electrodes are immersed into a solution of dye, and kept at room temperature. The dye-adsorbed TiO₂ electrode and thermally platinized counter electrode are assembled into a sealed sandwich type cell with a gap of a hot-melt ionomer film. The internal space is filled with electrolyte comprising I₂, N-methylbenzimidazole iodide, 1-methyl-3-propylimidazolium iodide and the ionic liquid prepared by the Example 1-2 or 2-2, using a vacuum pump to produce the DSSC device. The electrolyte-injecting hole made with a sandblasting drill on the counter electrode glass substrate is sealed. The characteristics of the DSSC device are measured.

### Example 6 : Use of the ionic liquid according to the present invention as solvent for separation of HCl from a mixture with C(O)F₂

A C(O)F₂/HCl gas mixture is prepared by photo-oxidation of CHF₂Cl. A crude gas mixture having the composition 45 % of C(O)F₂, 34 % of HCl, 6 % of O₂ and further reaction components (starting materials, etc.) is passed at a flow rate of 0.8 8 mol/h and slightly elevated pressure through a wash bottle containing 115 g (0.49 mol) of 1,2-dimethyl-3-(trifluoromethyl)-1H-pyrazolium triflate prepared by the Example 1-2 or 2-2. To obtain hydrolysis-sensitive components (e.g. C(O)F₂) of a gas mixture, it is advantageous to exclude or minimize moisture in the apparatus and in the ionic liquid. This is achieved by flushing the apparatus with dry nitrogen and evacuating it while heating the ionic liquid. After commencement of the experiment, the gas composition is measured by means of gas chromatography.

## Claims

1. An ionic liquid comprising at least one pyrazolium cation which is substituted by at least one fluorine atom, and at least one anion (An).

2. The ionic liquid according to Claim 1, wherein the pyrazolium cation has the formula (A) below : 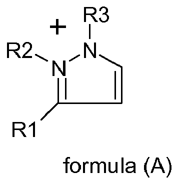 wherein, R1 is a fluorinated alkyl group preferably selected from CF₃, CF₂H and CF₂Cl, and R2 and R3 are independently hydrogen or an optionally fluorinated C 1 to C6 alkyl group, preferably selected from methyl group, ethyl group and butyl group.

3. The ionic liquid according to Claim 1 or 2, wherein the anion An is selected from the group consisting of halides, BF₄⁻, B(CN)₄⁻, CH₃BF₃⁻, CH₂CHBF₃⁻ , CF₃BF₃⁻, PF₆⁻, CF₃CO₂⁻ , CF₃SO₃⁻, (CF₃SO₂)₂N⁻, (CF₃SO)(CF₃CO)N⁻, (CN)₂N⁻, C(CN)₃⁻, SCN⁻, SeCN⁻, CuCl₂⁻, AlCl₄⁻, (CF₃)₂CFSO₃⁻, P(CN)ₚX₆₋ₚ (wherein p is an integer selected from between 1 and 6), (CF₃)₂CFCO₂⁻, and any combination thereof.

4. The ionic liquid according to any one of Claims 1 to 3, wherein R1 is CF₃, and each of R2 and R3 are a methyl group.

5. A method of manufacturing an ionic liquid comprising at least one pyrazolium cation which is substituted by at least one fluorine atom, and at least one anion (An), according to any one of Claims 1 to 4, comprising a step of contacting a compound having the formula Alk-An, wherein Alk is an optionally fluorinated C1 to C6 alkyl group with a solution of the pyrazole compound having formula (a-1) or formula (a-2) below, or the mixture thereof: 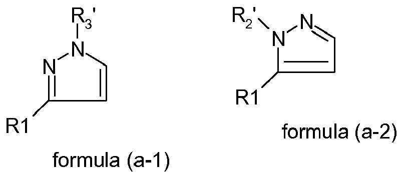 wherein, R1 is a fluorinated alkyl group preferably selected from CF₃, CF₂H and CF₂Cl, and R2' and R3' are independently hydrogen or an optionally fluorinated C1 to C6 alkyl group, preferably selected from hydrogen, methyl group, ethyl group and butyl group.

6. The method according to Claim 5, wherein R3' is hydrogen, further comprising a step of contacting alkyl halide with the pyrazole compound, before conducting the step of contacting Alk-An with a solution of the pyrazole compound having formula (a-1).

7. An electrolyte comprising the ionic liquid according to any one of Claims 1 to 4.

8. The electrolyte according to Claim 7, further comprising at least one conducting salt, preferably selected from lithium salts.

9. The electrolyte according to Claim 8, wherein the conducting salt is selected from the group consisting of LiPF₆, LiPO₂F₂, LiBF₄, LiB(CN)₄, LiAsF₆, LiClO₄, LiBOB (lithium bisoxalatoborate), LiFSI (lithium bis(fluorosulfonyl)imidide), Li[N(SO₂CₙF₂ₙ₊₁)(SO₂CmF₂ₘ₊₁)] (wherein n and m are independently integers selected from between 1 and 10, such as LiTFSI (lithium bis(trifluoromethylsulfonyl)imidide) or LiN(CF₃SO₂)₂), LiBeti (lithium bis(perfluoroethylsulfonyl)imidide), LiODBF (LiBF₂C₂O₄), LiB(C₆H₅), LiCF₃SO₃, LiC(CF₃SO₂)₃, and any combination thereof.

10. The electrolyte according to Claim 7, comprising at least one further ionic liquid with iodide as anion.

11. An electrochemical and/or optoelectronic device preferably selected from batteries and/or solar cells, comprising the electrolyte according to any one of Claims 7 to 10.

12. The electrochemical and/or optoelectronic device according to Claim 11, being a lithium ion battery.

13. The electrochemical and/or optoelectronic device according to Claim 11, being a dye-sensitized solar cell (DSSC) or an organic photovoltaic (OPV).

14. Use of an ionic liquid according to anyone of claims 1 to 4 as solvent, catalyst, electrically conducting fluid, sealant, surfactant or dispersing agent.

15. Use according to claim 14, as solvent for separating hydrogen halide from a gas mixture containing hydrogen halide and at least one other gas, preferably COF₂.
